# EUROPEAN PATENT APPLICATION

(11) **EP 1 627 630 A1**
(43) Date of publication of application: **22.02.2006**
(21) Application number: 04723771.4
(22) Date of filing: 26.03.2004
(51) Int. Cl.: A61K 9/20

(54) **DIVIDABLE TABLET**

(30) Priority: 28.03.2003 JP 2003092745
(71) Applicant: KYOWA HAKKO KOGYO CO., LTD., Chiyoda-ku, Tokyo 100-8185 (JP)
(72) Inventor: MIYABE, Junichi, Sunto-gun, Shizuoka 411-8731 (JP); MURAI, Kouji, Sunto-gun, Shizuoka 411-8731 (JP); GOTO, Tomohiko, New district, Su Zhou Jiang Su Province (CN); UENO, Yasuhiko, Sunto-gun, Shizuoka 411-8731 (JP)
(74) Representative: Vossius & Partner
(86) International application number: PCT/JP2004/004369
(87) International publication number: WO 2004/087110

(57) **Abstract**

An object of the present invention is to provide a dividable tablet that is easily and accurately divided without any particular limitation on dividing method thereof and has superior strength.

That is, the present invention provides a dividable tablet whose lower surface is a curved surface gradually rising from the central part exactly below the dividing line and whose upper surface is divided into two or more surfaces by a dividing line, wherein, in a side view of the tablet seen from the extended line of the dividing line, in a virtual triangle connecting the center of the lower surface and any two points on each of the two or more upper surfaces having the farthest distance from a tangent line to the center of the lower surface, the angle at the center of the lower surface is greater than or equal to 70°, and the ratio c/d of the distance (c) between any one of the points on each of the two or more upper surfaces having the farthest distance from the tangent line to the center of the lower surface and the tangent line to the center of the lower surface and the distance (d) between the lowest part of the dividing line and the center of the lower surface is 1/0.70 ≤ c/d ≤ 1/0.40.

## Description

### Technical Field

The present invention relates to a dividable tablet.

### Background Art

In pharmaceutical products and the like, tablets have been widely used as a dosage form having high versatility. Among them, dividable tablet is a tablet that is divided by pharmacists when preparing in accordance with the dosage or by the patients themselves when taking the medication, and has been developed to optimize the management of the dosage for individual patient and to enhance convenience of preparing.

Such dividable tablets generally provide a dividing line (dividing groove) for dividing the tablet. An example of the dividable tablet includes those disclosed in, for example, JP-U-1996-001012, JP-U-1995-019339, JP-A-1996-277217, US4353887 and US4824677, and in all cases, a method in which the pharmacist or the patient opens (or closes) the dividing line while holding the tablet with both hands, and a method in which the tablet is pressed along the dividing line or the exact back of the dividing line with the edge of a spoon and the like on a hard flat surface such as a table, are used as the dividing operation. For precise dividing, the force for dividing must be applied evenly, but the accuracy thereof cannot be expected in a manual operation. Thus, machine for automatically dividing the tablet has been developed, but is difficult to be used in time of urgency, or when the patient desires to divide it on his/her own. In JP-U-1996-001012, and JP-A-1996-277218, the shape of the tablet is designed to enhance the dividing precision, but the dividable tablet, which is recently becoming small, is difficult to divide with such shape. Further, the tablet is preferably divided from the outside the packaging such as press through pack (hereinafter referred to as PTP) (e.g., vinyl chloride/vinylidene chloride composite sheet) so as to divide and take out the tablet from the container without directly touching it with hand in order to maintain good hygiene, but the dividable tablets disclosed in the above articles are extremely difficult to divide from the outside the packaging.

Therefore, in JP-Y-1965-003680, JP-B-3015677, GB1387643, and WO99/51207, dividing is easily performed by pressing the dividable tablet sandwiched between two flat plates. Further, dividing is also possible by pressing the dividable tablet with the dividing line facing downward on the flat surface with the fingers and the like. Dividing is even possible from the outside the packaging such as PTP as long as the dividing line is facing downward. In such dividable tablet, when the dividable tablet is sandwiched between two flat plates, contacting points of the dividable tablet and the flat surface exist on both sides of the dividing line, and further, one contacting point exists at the exact back of the dividing line on the surface opposite the dividing line, and thus three force-appealed points are created thereby allowing the dividing operation. By using such dividing method, the dividing operation is not only facilitated but also applicable for small tablets.

Moreover, in WO99/51207, dividing is easily performed by pressing dividable tablet with the dividing line facing either downward or upward on the flat surface with the fingers and the like. Further, dividing is even possible for the dividing line facing either downward or upward from the outside the packaging such as PTP. The dividable tablet is characterized in that when the dividing line is arranged facing downward on the flat surface, one contacting point of the dividable tablet and the flat surface exists on both sides of the dividing line, and surfaces from the contacting point to the dividing line are a convex surface in the downward direction. In such dividable tablet, even if pressed from above with the dividing line facing upward on the flat surface, as the surfaces from the contacting point to the lowest part of the dividing line are a convex surface in the upward direction, such convex surfaces and the surface of the finger or the container of the PTP newly form an appropriate contacting point, and further, contacts the flat surface at one point on the exact back of the dividing line on the surface opposite the dividing line, and thus three force-appealed points are created, thereby allowing the dividing operation. The dividable tablet of JP-Y-1965-003680, JP-B-3015677, and GB1387643 is less likely to divide as it is difficult to appropriately contact both sides of the dividing line when pressed from above with the dividing line facing upward. Due to the difficulty in dividing, the dividing precision lowers, and thus differs from the dividable tablet of WO99/51207.

GB1387643 describes an angle of dividing line to facilitate dividing, and a distance from the lowest part of the dividing line to the lower surface with respect to a distance from the upper surface to the lower surface. However, as the center angle of the dividing line is required to be large, the dividing precision lowers. Further, although the distance from the lowest part of the dividing line to the lower surface is required to be small with respect to the distance from the upper surface to the lower surface, this weakens the hardness of the tablet.

In JP-Y-1965-003680, JP-B-3015677 and WO99/51207, the size and angle of the dividing line are described to improve easy dividing and enhance dividing precision, however, the shape other than such part is not described. When coating such dividable tablet, the shape of the upper surface or the lower surface is explained in an aim to prevent faults such as twinning, but no explanation is made whether such shape influences easy dividing or dividing precision. This is because, as the shape of the dividable tablet is a unique polyhedron, the shape or the size of each surface and the positional relationship between the side and the angle (including three force-appealed points) must be intricately combined and investigated, which was difficult beyond the scope of the ordinary optimal investigation made by those skilled in the art.

That is, a dividable tablet that is easily divided by being sandwiched between two flat plates and then pressed, that is easily divided by being pressed from above with the dividing line facing downward on the flat surface with fingers and the like, and further, that is divided through a similar method even from the outside the packaging such as PTP, and preferably, that is easily divided by being pressed with finger and the like from above regardless of whether the dividing line is arranged facing downward or upward on the flat surface, the tablet, being easily divided and having high dividing precision as well as hardness has not been specifically disclosed. Although such disclosure of the technique has been keenly desired, it has not been made apparent due to the unique polyhedron shape of the dividable tablet.

### Disclosure of the Invention

The object of the present invention is to provide a dividable tablet that is divided by being sandwiched between two flat plates, followed by pressing, that is divided by being pressed with fingers and the like with the dividing line facing downward on the flat surface, that is divided through similar methods even from the outside the packaging such as PTP, and preferably, that is divided regardless of whether the upper or the lower surface of the tablet is facing upwards, the dividable tablet being easily and accurately divided and having superior strength.

The inventors of the present invention have studied intensively to achieve the above object, and have found that by defining not only the angle of the dividing line, but also the entire shape as below, the dividable tablet that is divided by being sandwiched between two flat plates and by pressing, that is divided by being pressed the dividable tablet with the dividing line facing downward on the flat surface with finger and the like, that is divided through similar methods even from the outside the packaging such as PTP, and preferably, that is divided regardless of whether the upper or the lower surface of the tablet is facing upwards, the dividable tablet being easily and accurately divided and having superior strength can be obtained. The inventors of the present invention have further studied and obtained the present invention.

That is, the present invention relates to:
(1) a dividable tablet whose lower surface is a curved surface gradually rising from a central part exactly below a dividing line and whose upper surface is divided into two or more surfaces by at least one dividing line formed by U-shaped groove, or V-shaped groove having a center angle of less than or equal to 105°, wherein, in a side view of the tablet seen from the extended line of the dividing line, in a virtual triangle connecting a center of the lower surface and any two points on each of the two or more upper surfaces having the farthest distance from a tangent line to the center of the lower surface, the angle at the center of the lower surface is greater than or equal to 70°, a ratio c/d of a distance (c) between any of the points on each of the two or more upper surfaces having the farthest distance from the tangent line to the center of the lower surface and the tangent line to the center of the lower surface and a distance (d) between a lowest part of the dividing line and the center of the lower surface is 1/0.70 s c/d s 1/0.40, and (i) when c/d is in the range of 1/0.60 s c/d s 1/0.40, the angle at the center of the lower surface of the corresponding virtual triangle is greater than or equal to 70°, (ii) when c/d is in the range of 1/0.65 ≤ c/d < 1/0.60, the angle at the center of the lower surface of the corresponding virtual triangle is greater than or equal to 75°, and (iii) when c/d is in the range of 1/0.70 ≤ c/d < 1/0.65, the angle at the center of the lower surface of the corresponding virtual triangle is greater than or equal to 70° and less than 75° , or greater than or equal to 78°;
(2) a dividable tablet whose lower surface is a curved surface gradually rising from a central part exactly below the dividing line and whose upper surface is divided into two or more surfaces by at least one dividing line formed by U-shaped groove, or V-shaped groove having a center angle of less than or equal to 105°, and a ridge line is arranged on each of a plurality of regions formed by the above-mentioned dividing line so as to surround the dividing line and to be positioned on the inner side than the edge of the tablet, wherein in a side view of the tablet seen from the extended line of the dividing line, in a virtual triangle connecting a center of the lower surface and any two points on each of the two or more upper surfaces having the farthest distance from a tangent line to the center of the lower surface, the angle at the center of the lower surface is greater than or equal to 70°, a ratio c/d of a distance (c) between any of the points on each of the two or more upper surfaces having the farthest distance from the tangent line to the center of the lower surface and the tangent line to the center of the lower surface and a distance (d) between the lowest part of the dividing line and the center of the lower surface is 1/0.70 s c/d ≤ 1/0.40, and (i) when c/d is in the range of 1/0.60 ≤ c/d ≤ 1/0.40, the angle at the center of the lower surface of the virtual triangle is greater than or equal to 70°, (ii) when c/d is in the range of 1/0.65 ≤ c/d < 1/0.60, the angle at the center of the lower surface of the virtual triangle is greater than or equal to 75°, and (iii) when c/d is in the range of 1/0.70 s c/d < 1/0.65, the angle at the center of the lower surface of the virtual triangle is greater than or equal to 70° and less than 75°, or greater than or equal to 78°; and
(3) a dividable tablet described in the above (1) or (2) wherein (i) the angle at the center of the lower surface of the virtual triangle is greater than or equal to 70° and c/d is 1/0.60 ≤ c/d ≤ 1/0.40, (ii) the angle at the center of the lower surface of the virtual triangle is greater than or equal to 75° and c/d is 1/0.65 ≤ c/d ≤ 1/0.40, or (iii) the angle at the center of the lower surface of the virtual triangle is greater than or equal to 78° and c/d is 1/0.70 ≤ c/d ≤ 1/0.40.

### Brief Description of the Drawings

Fig. 1 is a schematic diagram showing a specific embodiment of a dividable tablet of the present invention, wherein A1 is a view seen from the upper surface, and B1 is a view of the side face seen from an extended line of a dividing line.
Fig. 2 is a schematic diagram showing another specific embodiment of a dividable tablet of the present invention, wherein A2 is a view seen from the upper surface, and B2 is a view of the side face seen from the extended line of the dividing line.
Fig. 3 is a side view showing a frame format of a phenomenon occurring in the dividable tablet when dividing the dividable tablet of the present invention by sandwiching the dividable tablet between two flat plates, followed by pressing.
Fig. 4 is a side view showing a frame format of a phenomenon occurring in the dividable tablet when applying force from top to bottom in the vertical direction and dividing a dividable tablet (40) of the present invention accommodated within an accommodating concave part (41) of an accommodating main body of PTP with an upper surface including a dividing line facing towards a sealing sheet (42) before taking the dividable tablet of the present invention out from the accommodating concave part.
Fig. 5 is a side view showing a frame format of a phenomenon occurring in the dividable tablet when applying force from top to bottom in the longitudinal direction and dividing a dividable tablet (40) of the present invention accommodated within the accommodating concave part (41) of an accommodating main body of PTP with an upper surface including a dividing line facing towards an accommodating main body (43) before taking the dividable tablet of the present invention out from the accommodating concave part.
Fig. 6 is a frame format view schematically showing a shape of the dividing line arranged on the dividable tablet according to the present invention.

In the figure, the reference 1 represents the lower surface, reference 2 represents the dividing line, reference 3 represents the central part (center), reference 6 represents the tangent line of the center 3 of the lower surface 1, reference 7 and 8 represent the point on the upper surface having the farthest distance from the tangent line 6 to the center 3 of the lower surface 1, reference 9 and 10 represent the ridge lines, reference 12 and 13 represent the border of the dividing line 2, reference R₁ represents the curvature radius of the lower surface 1, reference a₁ represents the central angle of the dividing line 2, reference b₁ represents the angle at the center 3 of the lower surface 1 of a virtual triangle connecting the center 3 of the lower surface 1 with each point 7 and 8, reference c₁ represents the distance between one of the points 7 and 8 and the tangent line 6 to the center 3 of the lower surface 1, reference d₁ represents the distance between the line 11 of the lowest part of the dividing line 2 and the center 3 of the lower surface 1, reference 18 represents the lower surface, reference 19 represents the dividing line, reference 20 represents the central part (center), reference 23 represents the tangent line to the center 20 of the lower surface 18, reference 24 and 25 represent the point on the upper surface having the farthest distance from the tangent line 23 to the center 20 of the lower surface 18, reference 26 and 27 represent the ridge lines, reference 29 and 30 represent the border of the dividing line 19, reference R₂ represents the curvature radius of the lower surface 18, reference S₂ represents the curvature radius of the upper surface, reference a₂ represents the central angle of the dividing line 19, reference b₂ represents the angle at the center 20 of the lower surface 18 of a virtual triangle connecting the center 20 of the lower surface 18 with each point 24 and 25, reference c₂ represents the distance between one of the points 24 and 25 and the tangent line 23 to the center 20 of the lower surface 18, reference d₂ represents the distance between the line 28 of the lowest part of the dividing line 19 and the center 20 of the lower surface 18, reference 40 represents the dividable tablet, reference 41 represents the accommodating concave part, reference 42 represents the sealing sheet, reference (a) represents the V-shaped groove, reference (b) represents the V-shaped groove in which the vicinity of the apex angle has a circular round shape, reference (c) represents the V-shaped groove in which the vicinity of the apex angle has one or more bended part, reference (d) represents the U-shaped groove, reference (e) represents the U-shaped groove having one or more bended part, reference (f) represents the V-shaped groove shape formed by intersecting the curves of convex shape, and reference 43 represents the press through pack.

### Best Mode for Carrying Out the Invention

The dividable tablet according to the present invention includes a dividable tablet having two or more dividing lines, but in the following description, a dividable tablet having one dividing line will be described by way of example.

The dividable tablet of the present invention is a dividable tablet whose lower surface is a curved surface gradually rising from the central part exactly below the dividing line and whose upper surface is divided into two or more surfaces by the dividing line formed by at least one U-shaped groove or at least one V-shaped groove having a central angle of less than or equal to 105°, and in the side view of the dividable tablet seen from the extended line of the dividing line, the dividable tablet has at least one point on the upper surface having the farthest distance from the tangent line to the center of the lower surface located on both sides of the dividing line, the upper surface of the dividable tablet contacting the flat surface at least one point on both sides of the dividing line when the dividable tablet is sandwiched between two flat plates, and the lower surface of the dividable tablet contacting the flat surface at the central part thus creating a total of three force-appealed points thereby becoming dividable.

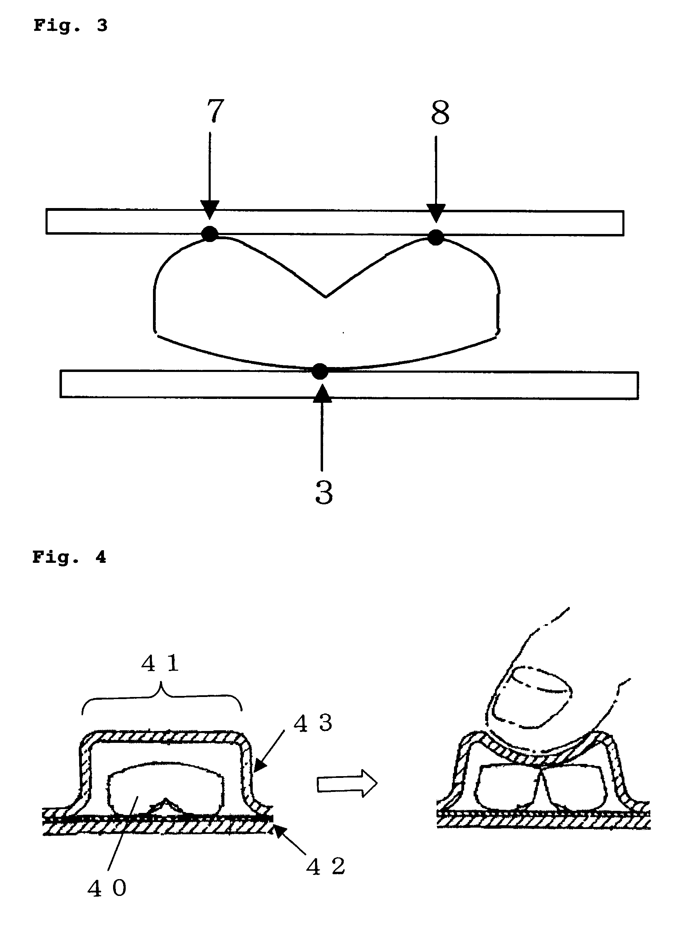

The dividable tablet of the present invention preferably includes the following embodiments. The dividable tablet of the present invention is a dividable tablet whose lower surface is a curved surface gradually rising from the central part exactly below the dividing line and whose upper surface is divided into two or more surfaces by the dividing line formed by at least one U-shaped groove or one V-shaped groove having a central angle of less than or equal to 105°, and in which ridge lines are arranged at each of a plurality of regions formed by the dividing line so as to surround the dividing line, and in the side view of the tablet seen from the extended line of the dividing line as shown in Fig. 3, the dividable tablet has at least one point each on the upper surface having the farthest distance from the tangent line to the center of the lower surface located on the ridge line of both sides of the dividing line (7 and 8 of Fig. 3), the upper surface of the dividable tablet contacting the flat surface at least one point each (7 and 8 of Fig. 3) on both sides of the dividing line when sandwiching the dividable tablet between two flat plates, and the lower surface of the dividable tablet contacting the flat surface at the central part (3 of Fig. 3) thus creating a total of three force-appealed points thereby becoming dividable. Usually, the distance between point 7 and point 3 is the same as the distance between the point 8 and point 3. Further, as a shape that gradually rises from the edge of the tablet towards the central part is adopted for the lower surface of the tablet in the relevant dividable tablet, when the dividable tablet (40 of Fig. 4) is accommodated in the accommodating concave part (41 of Fig. 4) of an accommodating main body of PTP with the upper surface including the dividing line facing the sealing sheet (42 of Fig. 4) as shown in Fig. 4, the tablet is easily divided into two parts by pressing the central part of convex surface on the lower surface of the tablet with finger from above the accommodating concave part of the accommodating main body of PTP.

The central part at the lower surface may be a plane, a line or a point.

In the present invention, the dividable tablet wherein the upper surface is divided into two surfaces by the dividing line formed by one U-shaped groove or one V-shaped groove having a central angle of less than or equal to 105°, and wherein the ridge lines are arranged at each of the two regions formed by the dividing line so as to surround the dividing line and so as to be positioned on the inner side than the edge of the tablet is given as a preferred embodiment.

In the dividable tablet of the present invention, the region around the ridge line is preferably configured with a convexly curved surface. That is, the region around the ridge line is preferably chamfered. By configuring the region around the ridge line into a convexly curved surface so as to remove the pointed edge portions from the upper surface of the tablet, the following advantages are obtained. When force is applied downward from above the tablet, the tablet is finely divided along the dividing line without producing chips. Further, in tableting process, pneumatic conveying, or carrying in PTP or bottle, the chips of the tablet are less likely to be produced. In the coating process of the dividable tablet, core erosion, edge chipping and the like are less likely to occur. Further, when accommodating the tablet into the accommodating concave part of PTP, a case where the sealing sheet is freely ripped by the tablet without pressing with finger and the like is less likely to occur.

In the dividable tablet of the present invention, the shape of the surface from the border line of the dividing line to the ridge line is preferably configured with a convexly curved surface at each of a plurality of regions formed by the dividing line on the upper surface of the tablet. More preferably, the upper surface is divided into two surfaces by the dividing line formed by one U-shaped groove or one V-shaped groove having a central angle of less than or equal to 105°, the ridge lines are arranged at each of the two regions formed by the dividing line so as to surround the dividing line and to be positioned on the inner side than the edge of the tablet, and the shape of the surface from each border line of the dividing line to each of the two ridge lines is configured with a convexly curved surface at each of the two regions formed by the dividing line on the upper surface of the tablet.

It is to be noted that the "convexly curved surface" refers to a gradual rising curved surface. More specifically, a case in which the curvature is continuously changed in the direction of the ridge line from the border line of the dividing line so as to gradually become a surface having a large curvature from the surface having a small curvature to obtain a gradual rising curved surface as a whole is provided. The "convexly curved surface" is more preferably a curved surface in which the curvature radius is greater than about 4 mm.

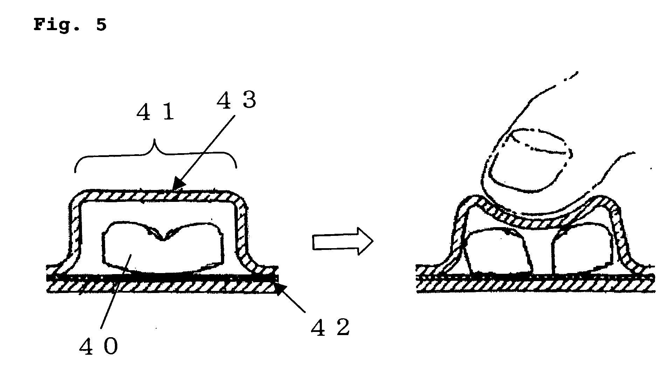

With the dividable tablet of the present invention having such shape, as shown in Fig. 4, not only in the case in which the dividable tablet is accommodated in the accommodating concave part of the accommodating main body of PTP with the upper surface including the dividing line facing the sealing sheet, but even in a case in which the dividable tablet is accommodated in the accommodating concave part (41 of Fig. 5) of PTP with the upper surface including the dividing line facing the accommodating main body (43 of Fig. 5) as shown in Fig.5, the dividable tablet is easily divided into two parts by pressing the accommodating concave part (41 of Fig. 5) of PTP with finger from above. As shown in Fig. 5, when the dividing of the dividable tablet is seen from the side, as each of the two regions formed by the dividing line on the upper surface of the tablet has a curved surface that faces each other match for the shape of the accommodating concave part squashed into a substantially V-shape or the finger, the force acts in a direction of opening the dividing line without applying great force on the finger. Thus, even when accommodated in the accommodating concave part of PTP with the upper surface including the dividing line of the tablet facing towards the accommodating main body, the tablet is easily divided into two parts. Therefore, the dividable tablet of the present embodiment has an advantage in that even when the tablet is accommodated in the accommodating concave part of the accommodating main body of PTP without regulating the orientation thereof, the tablet is easily divided into two parts before being taken out from the PTP. The dividable tablet of the present embodiment has two convex surfaces on the upper surface and a convex surface on the lower surface, and thus the tablets do not surface contact each other but point contact each other. Thus, an advantage in that twinning is less likely to occur in the coating process is obtained. Further, the dividable tablet of the present invention is easily divided by being pressed down with finger and the like when the tablet is taken out from the PTP regardless of whether the dividing line is facing downward or upward on the flat surface. When being pressed with finger and the like with the dividing line facing upward on the flat surface, the force-appealed point is sometimes shifted from the point on the upper surface having the farthest distance from the tangent line to the center of the lower surface, but even in such event, if the surface from the contacting point to the lowest part of the dividing line has a convex shape in the upward direction, the relevant surface and the finger newly form an appropriate contacting point, and thus further contacts the flat surface at the central part exactly back of the dividing line on the surface opposite the dividing line thus creating three force-appealed points and becoming dividable.

In the present invention, as another embodiment of a tablet that can be divided regardless of whether the upper or the lower surface of the dividable tablet faces upwards, a dividable tablet in which a line connecting to the dividing line with each point on the two upper surfaces having the farthest distance from the tangent line to the center of the lower surface as the starting point is a linear line forming an angle of less than or equal to about 30° with the tangent line to the center of the lower surface is provided. In this case, the part from the ridge line to the dividing line may be a linear line, a concavity curved line, or a wavy curved line.

In the dividable tablet of any of the above embodiments, the dividing line arranged on the upper surface may be a V-shaped groove or a U-shaped groove.

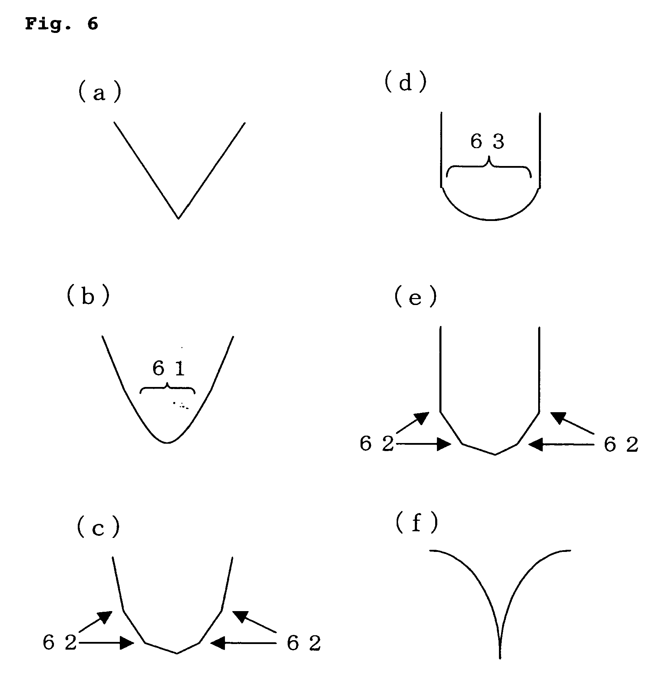

In the case of the V-shaped groove, the center angle thereof is less than or equal to 105°, preferably less than or equal to 90°, and more preferably less than or equal to 70°. It is to be noted that the "V-shaped groove" includes a substantially V-shaped groove. The "substantially V-shaped groove" includes a case in which the vicinity of the apex angle of the V-shaped groove has a shape formed so as to be round with a curved line or is a shape similar to the vicinity of the vertex of a parabola (quadratic curve), a case in which the vicinity of the apex angle of the V-shaped groove is a polygon shape having a bended part and the like. More specifically, the V-shaped groove in the present invention may be a V-shaped groove of a shape shown in Fig. 6(a), a V-shaped groove of a shape in which the vicinity of the apex angle has a rounded part 61 as shown in Fig. 6(b), or a V-shaped groove in which the vicinity of the apex angle has a polygon shape having one or more bended part 62 as shown in Fig. 6(c).

The U-shaped groove includes a substantially U-shaped groove. The "substantially U-shaped groove" includes, for example, a case in which a round curved line portion of the U-shaped groove is formed into a polygon shape having a bended part and the like. More specifically, the U-shaped groove in the present invention may be a U-shaped groove of a shape shown in Fig. 6(d), or a U-shaped groove of a shape in which the lower part is not formed into a smooth curved line (region 63 shown in Fig. 6(d)) but formed into a polygon shape having one or more bended part 62 as shown in Fig. 6(e).

The V-shaped groove includes a case of a V-shaped groove formed by intersecting the convexity curved lines with each other as shown in Fig. 6(f), and includes a case in which the inflexion point does not exist at the end of the dividing line, and the end of the dividing line and the ridge line are the same.

In the dividable tablet of the present invention, with respect to the side view of the tablet seen from the extended line of the dividing line and with regards to a virtual triangle (hereinafter simply referred to as "virtual triangle") formed by connecting the center of the lower surface and any two points on each of the two or more upper surfaces having the farthest distance from the tangent line to the center of the lower surface, the angle at the center of the lower surface is preferably greater than or equal to about 70°, more preferably greater than or equal to 75°, and further most preferably greater than or equal to about 78°. Most preferably, when the ratio c/d of the distance (c) between any of the points on each of the two or more upper surfaces having the farthest distance from the tangent line to the center of the lower surface and the tangent line to the center of the lower surface and the distance (d) between the lowest part of the dividing line and the center of the lower surface is in the range of (i) 1/0.60 ≤ c/d ≤ 1/0.40, the angle at the center of the lower surface of the virtual triangle is greater than or equal to 70°, (ii) when c/d is in the range of 1/0.65 ≤ c/d < 1/0.60, the angle at the center of the lower surface of the virtual triangle is greater than or equal to 75°, and (iii) when in the range of 1/0.70 ≤ c/d < 1/0.65, the angle at the center of the lower surface of the virtual triangle is greater than or equal to 70° and less than 75°, or greater than or equal to 78°.

For the purpose of having the angle at the center of the lower surface of the above mentioned virtual triangle to be greater than or equal to 70° and the center angle of the dividing line to be less than or equal to 105°, the surface from the ridge line on the upper surface to the central line of the dividing line is preferably made into a curved surface, or into a polyhedron that inflects at the border of the dividing line.

In order to more easily and more accurately divide the dividable tablet of the present invention, the ratio c/d between the distance (c) between any of the point on each of the two or more upper surfaces having the farthest distance from the tangent line to the center of the lower surface and the tangent line to the center of the lower surface and the distance (d) between the lowest part of the dividing line and the center of the lower surface is preferably within the following range in relation to the angle at the center of the lower surface of the virtual triangle. If the angle at the center of the lower surface of the virtual triangle is greater than or equal to 70° and less than 75°, the ratio c/d is more preferably made to about 1/0.60 ≤ c/d ≤ 1/0.40, if the angle at the center of the lower surface of the virtual triangle is greater than or equal to 75° and less than 78°, the ratio c/d is more preferably made to about 1/0.65 ≤ c/d ≤ 1/0.40, and if the angle at the center of the lower surface of the virtual triangle is greater than or equal to 78°, the ratio c/d is more preferably made to about 1/0.70 s c/d s 1/0.40. Further, the ratio c/d is more preferably made to about 1/0.60 ≤ c/d ≤ 1/0.50 regardless of the angle at the center of the lower surface of the virtual triangle. If the ratio c/d is 1/0.60 ≤ c/d s 1/0.50, the angle at the center of the lower surface of the virtual triangle is more preferably made to greater than or equal to 78°.

In the dividable tablet of the present invention, the contour when seen from the upper surface may be an arbitrary shape such as a circle, an oval, a polyhedron and a star-shape, however, it is preferably a circle or an oval. The size of the tablet may be large or small, and the diameter may be, for example, between about 6 mm to 12 mm, and for smaller ones, between about 3 mm to 6 mm.

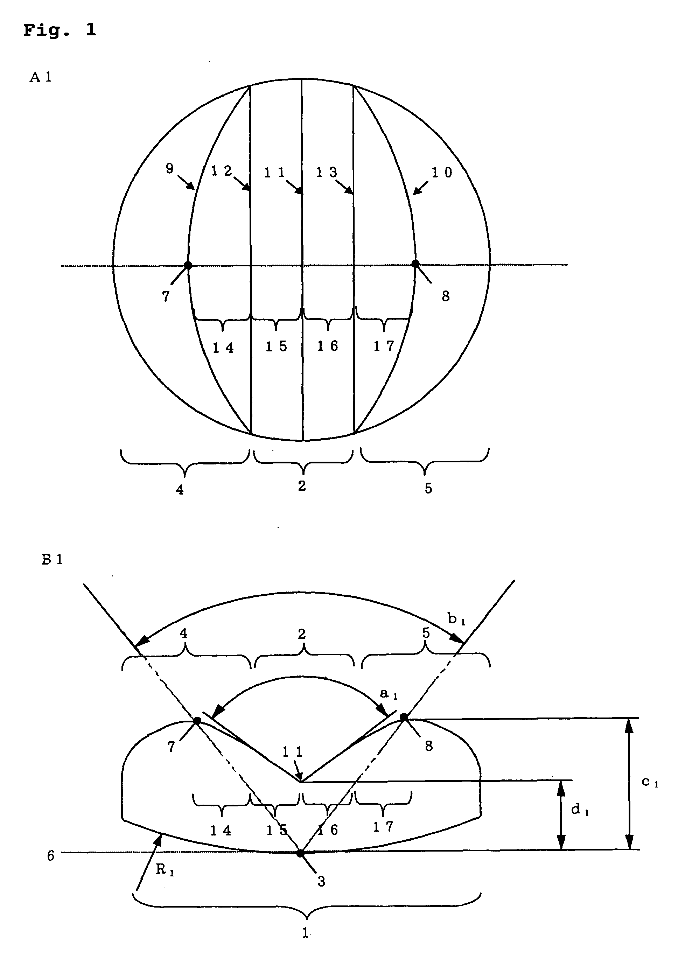

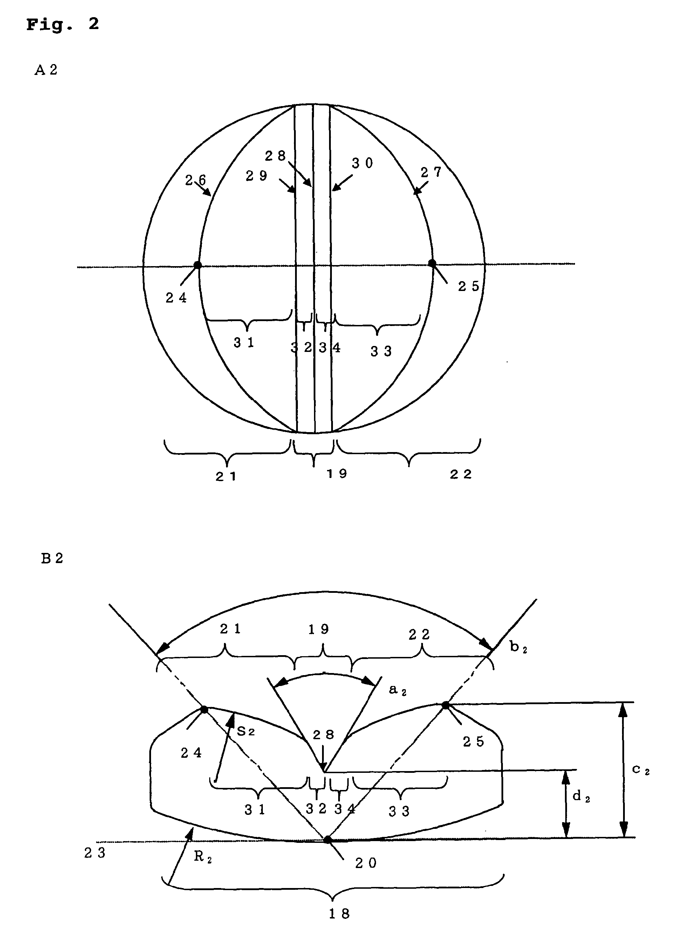

In the dividable tablet of the present invention, the thickness (c₁ value in Fig. 1, c₂ value in Fig. 2) with respect to the diameter of the tablet is preferably greater than or equal to 0.25 and more preferably greater than or equal to 0.40 relative to the diameter as 1 to enhance the mechanical strength of the entire tablet.

Further, in order to more easily and more accurately divide the dividable tablet of the present invention, the lower surface of the dividable tablet is preferably a curved surface having the curvature radius of greater than 7 mm, more preferably a curved surface having the curvature radius of greater than 9 mm, and most preferably a curved surface having the curvature radius of greater than 11 mm.

Preferably, the dividable tablet of the present invention takes one of the shapes described in (a) to (d) below.
(a) In the dividable tablet of the present invention, the distance from the lowest part of the dividing line to the center of the lower surface is preferably shorter than the distance from the point at the upper end of the edge of the tablet to the tangent line to the center of the lower surface. In this case, the dividing line of the tablet is formed deeper than the dividing line of a normal dividable tablet, and thus can be divided nicely along the dividing line.
(b) In the dividable tablet of the present invention, each border line of the dividing line is preferably arranged at a position above the upper end of the edge of the tablet. That is, the distance from the point on the border line of the dividing line to the tangent line to the center of the lower surface is preferably longer than the distance from the point at the upper end of the edge of the tablet to the tangent line of the center of the lower surface. It is to be noted that, normally, two "the border line of the dividing line" are present for one dividing line.
   Normally, in the dividable tablet, dividing into two parts is easily carried out if the dividing line is formed deep in the longitudinal direction of the tablet from the upper surface of the tablet so that the depth of the dividing line is deep, but this weakens the mechanical strength at the region where the dividing line is arranged, and the tablet is freely and easily divided into two parts although not intended to be divided. According to the above configuration of the invention, the depth of the dividing line is made deep without relatively lowering the mechanical strength of the tablet at the region where the dividing line is arranged, and thus can be easily divided into two parts, and, the tablet is substantially prevented from being freely divided into two parts when not being intended to be divided.
(c) In the dividable tablet of the present invention, the shape from each of a plurality of, preferably two, ridge lines on the upper surface of the tablet to the upper end of the edge of the tablet is preferably a sharp inclined surface. According to such configuration, the shape from the border of the dividing line to the ridge line becomes a gradual rising curved line compared to the shape from each ridge line to the edge of the tablet at the upper surface of the tablet, and thus when force is applied to the upper surface of the tablet from above in the downward direction, the force is easily applied in a direction of opening the dividing line, thereby allowing more superior dividing along the dividing line with a smaller force.
   The "sharp inclined surface" is preferably a linear line or a convexity curved line in the side view of the tablet seen from the extended line of the dividing line of the dividable tablet. The decision of whether to have the shape of the "sharp inclined surface" to be a linear line or a convexity curved line is determined in view of the design of the tablet. That is, on the basis of medicinal effects, indications, efficacies and the like of the dividable tablet, the shape of the tablet is formed so that the shape of the tablet makes an impression on the physicians, pharmacists, patients and the like.
(d) In the dividable tablet of the present invention, chamfering is preferably performed in the vicinity of the part where the dividing line intersects with the edge of the tablet. Further, such vicinity is more preferably chamfered into two convex surfaces formed so as to sandwich the dividing line. According to such configuration, chipping is less likely to occur in the tablet.

A specific embodiment of the present invention is shown in Fig. 1. In this figure, A1 is a schematic view seen from the upper surface, and B1 is a schematic view of the side seen from the extended line of the dividing line.

In the dividable tablet of Fig. 1, the lower surface 1 is a curved surface gradually rising from the central part 3 exactly below the dividing line 2. It is to be noted that in the embodiment shown in Fig. 1, the "central part of the lower surface" is a point and, is to be indicated the point same as the "center of the lower surface", both are denoted with reference 3. However, the "central part of the lower surface" is not limited to a point, and may be a line or a surface, as noted above. The upper surface is divided into surfaces 4 and 5 by the dividing line 2, and ridge lines 9 and 10 are formed on the surfaces 4 and 5 with the dividing line in between. The points 7 and 8 on the upper surface having the farthest distance from the tangent line 6 to the center 3 of the lower surface 1 are located on the ridge lines 9 and 10, respectively, on both sides (surfaces 4 and 5) of the dividing line 2. Two surfaces of surfaces 14, 15 and surfaces 16, 17 each inflected at lines 12 and 13 are formed from the ridge lines 9 and 10 to the line 11 at the lowest part of the dividing line 2.

Further, with the curvature radius of the lower surface 1 shown as R₁, the center angle of the dividing line 2 shown as a₁, the angle at the center 3 of the lower surface 1 of the virtual triangle connecting the center 3 of the lower surface 1 and each point 7 and 8 on the surfaces 4 and 5 shown as b₁, the distance between one of the points 7 and 8 and the tangent line 6 to the center 3 of the lower surface 1 shown as c₁, and the distance between the line 11 at the lowest part of the dividing line 2 and the center 3 of the lower surface 1 shown as d₁, R₁ is preferably greater than or equal to about 7 mm, more preferably greater than or equal to about 9 mm, and most preferably greater than or equal to about 11 mm, a₁ is preferably less than or equal to about 105°, more preferably less than or equal to about 90°, most preferably less than or equal to about 70°, and b₁ is preferably greater than or equal to about 70°, more preferably greater than or equal to about 75°, most preferably greater than or equal to about 78°, where when b₁ is greater than or equal to 70° and less than 75°, the ratio of c₁/d₁ is about 1/0.60 ≤ c₁/d₁ ≤ 1/0.40, when b₁ is greater than or equal to 75° and less than 78°, the ratio of c₁/d₁ is about 1/0.65 ≤ c₁/d₁ ≤ 1/0.40, and when b₁ is greater than or equal to 78°, the ratio c₁/d₁ of c₁ and d₁ is about 1/0.70 ≤ c₁/d₁ ≤ 1/0.40. Usually, it is to be noted that the distance between point 7 and the tangent line 6 is the same as the distance between the point 8 and the tangent line 6.

Further, one specific embodiment of when the dividable tablet of the present invention is a tablet that is dividable regardless of whether the upper or the lower surface is facing upward is shown in Fig. 2. In this figure, A2 is a schematic view seen from the upper surface, and B2 is a schematic view of the side face seen from the extended line of the dividing line.

In the dividable tablet of Fig. 2, the lower surface 18 is a curved surface gradually rising from the central part 20 exactly below the dividing line 19. It is to be noted that in the embodiment shown in Fig. 2, the "central part of the lower surface" is a point, and is to be indicated the point same as the "center of the lower surface", both are denoted with the reference 20. The upper surface is divided into surfaces 21 and 22 by a V-shaped groove 19, and ridge lines 26 and 27 are arranged on the surfaces 21 and 22 with the dividing line in between. The points 24 and 25 on the upper surface having the farthest distance from the tangent line 23 to the center 20 of the lower surface 18 are located on the ridge lines 26 and 27, respectively, on both sides (surface 21 and 22) of the dividing line 19. Two surfaces of surfaces 31, 32 and surfaces 33, 34 each inflected by the lines 29 and 30 are formed from the ridge lines 26 and 27 to the line 28 at the lowest part of the dividing line 19.

Further, with the curvature radius of the lower surface 18 shown as R₂, the curvature radius of the surfaces 31 and 33 as S₂, the center angle of the dividing line 19 shown as a₂, the angle at the center 20 of the lower surface 18 of the virtual triangle connecting the center 20 of the lower surface 18 and each point 24 and 25 on the surfaces 21 and 22 shown as b₂, the distance between one of the points 24 and 25 and the tangent line 23 to the center 20 of the lower surface 18 shown as c₂, and the distance between the line 28 at the lowest part of the dividing line 19 and the center 20 of the lower surface shown as d₂, R₂ is preferably greater than or equal to about 7 mm, more preferably greater than or equal to about 9 mm, and most preferably greater than or equal to about 11 mm, S₂ is preferably greater than or equal to about 4 mm, a₂ is preferably less than or equal to about 105°, more preferably less than or equal to about 90°, most preferably less than or equal to about 70°, and b₂ is preferably greater than or equal to about 70°, more preferably greater than or equal to about 75°, most preferably greater than or equal to about 78°, where when b₂ is greater than or equal to 70° and less than 75°, the ratio c₂/d₂ of c₂ and d₂ is about 1/0.60 ≤ c₂/d₂ ≤ 1/0.40, when b₂ is greater than or equal to 75° and less than 78°, the ratio c₂/d₂ of c₂ and d₂ is about 1/0.65 ≤ c₂/d₂ ≤ 1/0.40, and when b₂ is greater than or equal to 78°, the ratio c₂/d₂ of c₂ and d₂ is about 1/0.70 ≤ c₂/d₂ ≤ 1/0.40. Usually, it is to be noted that the distance between point 24 and the tangent line 23 is the same as the distance between the point 25 and the tangent line 23.

The dividable tablet of the present invention simply needs to be formed into the above mentioned shape, and has no limitation regarding the ingredients to be contained therein. Examples of an active ingredient that may be contained in the dividable tablet of the present invention include one or, two or more active ingredients selected from central nervous system drugs, peripheral nervous system drugs, drugs for circulation organs, drugs for digestive organs, hormones, drugs for urogenital organs, drugs for blood and body fluid, metabolic drugs, antitumor agents, antiallergic drugs, antibiotics, chemotherapeutic agents, vitamins, diagnostic agents, narcotics/hallucinogens and the like. Specific examples of an active ingredient include one or, two or more active ingredients selected from the group consisting of acetylspiramycin, amoxicillin, ethyl icosapentate, itraconazole, oxatamide, olopatadine, glybuzole, glutathione, ketophenylbutazone, cobamamide, cisapride, todralazine, tropisetron, domperidone, valproic acid, pyridoxal, fluorouracil, flunarizine, flurazepam, benidipine, minocycline, mebendazole, medroxyprogesterone, ubidecarenone, levodopa and salt thereof. The active ingredient is generally contained within a range of greater than or equal to about 0.01 parts by weight and less than or equal to 90 parts by weight in one tablet (weight of one tablet being 100 parts by weight). The dividable tablet of the present invention is preferably used as medicines, but is also preferably used in foods, cosmetics, agricultural chemicals, and animal drugs.

The dividable tablet of the present invention may contain various additives used in the production of general formulation, and the additive amount thereof is the amount used in the production of the general formulation. Examples of additives include excipients, binders, disintegrators, lubricants, flavors, foaming agents, sweeteners, perfumes, coloring agents, stabilizers, solubilizers and the like.

Examples of the excipients include sugars such as lactose, mannitol, sucrose and glucose; starches such as potato starch, corn starch and partial pregelatinized starch; inorganic salts such as precipitated calcium carbonate, calcium phosphate and sodium chloride; microcrystalline celluloses; and the like, and in addition, those that are known may also be used without any limitations. The excipients are generally contained in a range of greater than or equal to about 10 parts by weight to less than or equal to 98 parts by weight in one tablet (weight of one tablet being 100 parts by weight).

Examples of the binders include partially hydrolyzed polyvinyl alcohol, hydroxylprocellulose, hydroxypropyl methylcellulose, polyvinylpyrrolidone, pullulan, methylcellulose, acacia, starch and the like, and in addition, those that are known may also be used without any limitations. The binders are generally contained in a range of greater than or equal to about 0.5 parts by weight to less than or equal to 15 parts by weight in one tablet (weight of one tablet being 100 parts by weight).

Examples of the disintegrators include crosspovidone, low substituted hydroxypropylcellulose, crosscarmellose sodium, carboxymethyl cellulose, calcium carboxymethyl cellulose, sodium carboxymethyl starch, partial pregelatinized starch, hydroxypropyl starch, corn starch, potato starch, microcrystalline cellulose, powdered cellulose and the like, and in addition, those that are known may also be used without any limitations. Among them, crosspovidone, low substituted hydroxypropylcellulose, or crosscarmellose sodium is preferably used. The disintegrators are generally contained in a range of greater than or equal to about 1 part by weight to less than or equal to 30 parts by weight in one tablet (weight of one tablet being 100 parts by weight).

Examples of the lubricants include magnesium stearate, calcium stearate, zinc stearate, stearic acid, talc crystallinum, hydrogenated vegetable oil, talc and the like, and among them, magnesium stearate or calcium stearate is preferable. The lubricants are contained in a range of greater than or equal to about 0.01 parts by weight and less than or equal to 5 parts by weight in one tablet (weight of one tablet being 100 parts by weight).

Examples of the flavors include an organic acid salt for flavoring such as monosodium glutamate, 5'-sodium inosinate, 5'-sodium guanylate, sodium aspartate and the like, and in addition, those that are known may also be used without any limitations.

Examples of the foaming agents include sodium bicarbonate, sodium carbonate and the like, and in addition, those that are known may also be used without any limitations.

Examples of the sweeteners includes aspartame, saccharin sodium, stevia and the like, and in addition, those that are known may also be used without any limitations.

Examples of the perfumes include lemon, orange, menthol and the like, and in addition, those that are known may also be used without any limitations.

Examples of the coloring agents include food colors such as Food Yellow No. 5, Food Red No.2, and Food Blue No. 2, Food Lake Colors, iron oxide and the like, and in addition, those that are known may also be used without any limitations.

Examples of the stabilizers include sodium edetate, tocopherol, cyclodextrin and the like, and in addition, those that are known may also be used without any limitations.

Examples of the solubilizers include polyethylene glycols, cyclodextrin, hydroxypropyl-cyclodextrin and polyvinylpyrrolidone (PVP) and the like, and in addition, those that are known may also be used without any limitations.

The dividable tablet according to the present invention is not particularly limited regarding its application and administration method.

The dividable tablet according to the present invention may be any one of orally taken tablet, effervescent tablet, prescribed tablet, chewable tablet, intraorally rapid disintegration tablet, troche, and oral tablet such as buccal and sublingual tablet.

The dividable tablet according to the present invention, other than a usual tablet or a coated tablet, may be a functional tablet such as multiple unit tablet, gradumet, wax matrix, resinate and spantab.

A method of manufacturing the dividable tablet of the present invention includes an indirect tableting method wherein active ingredients and various additives are granulated through each method of, for example, fluidized bed granulation, agitation granulation, rolling granulation, rolling fluidized granulation, extruding granulation, mixed with the lubricant as desired to obtain a powder for tableting, and then tableted with a tableting machine, which is itself known and is preferably a rotary tableting machine or a single shot tableting machine excellent in the productivity. Further, there is also known a direct tableting method in which a mixture of active ingredients and various additives acts as a powder for tableting, and is tableted with a tableting machine, which is itself known and is preferably, a rotary tableting machine or a single shot tableting machine excellent in the productivity. Further, lubricants may be applied in advance to the punch and die of the tableting machine during compression molding, and then compression molding may be performed without mixing the lubricant with the granules in the indirect tableting method or the mixture in the direct tableting method.

The dividable tablet of the present invention may be film coated as desired. The coating performed on the tablet of the present invention may be that normally used in coating the tablet. An example of a film forming polymer used to form the film coating includes a cellulose polymer. The cellulose polymer preferably has a molecular weight of 7,500 to 120,000 and a viscosity of 1.5 to 20 mm²/s (25C°). For example, cellulose ether polymer which is one of cellulose polymer includes hydroxylpropyl methylcellulose (HPMC), hydroxylpropyl cellulose, methylcellulose, ethylcellulose and the like, among which HPMC is preferable. TC-5 (Japanese Pharmacoepia hydroxypropyl methylcellulose 2910), for example, is used as HPMC.

The method of dividing the dividable tablet of the present invention is not particularly limited. For example, the dividable tablet of the present invention is easily divided by being sandwiched between two flat plates, followed by pressing, as shown in Fig. 3. Preferably, it is divided by pressing with finger and the like with the dividing line facing downward on the flat surface. Preferably, it is also divided by pressing with finger and the like with the dividing line facing upward on the flat surface. Further, even if the dividable tablet of the present invention is accommodated in the PTP, the dividable tablet is divided through the above mentioned method. The dividable tablet of the present invention is also divided through methods such as, a method of holding the tablet with both hands and then opening (or closing) the dividing line and a method of pressing along the dividing line or the exact back of the dividing line with the edge of the spoon and the like on a hard flat surface such as a table.

The present invention will be specifically explained by way of examples, but these examples are not intended to limit the present invention.

### Examples

### Example 1

In a fluidized bed granulator (Freund Co. , Ltd. : Flow Coater FLO-15), 66.6% by weight of lactose (DMV corporation: 200 M) and 29.4% by weight of corn starch (Nihon Shokuhin Kako Co., Ltd.: Nihon Shokuhin corn starch W) were placed, an aqueous solution of 10% by weight of partially hydrolyzed polyvinyl alcohol (Nippon Synthetic Chemical Industry Co. , Ltd.: Gohsenol EG-05) was prepared as a binder and sprayed onto the above mixture, then granulated and dried to obtain dried powder. The dried powder contained 3% by weight of binder.

Next, the dried powder prepared as above was size reduced with a crusher (Tokuju Co. Ltd: Rundell Mill RM-I), and 1% by weight of lubricant (magnesium stearate:Sakai Chemical Industry Co. Ltd) were added to the resulting granules to mix for five minutes in a blender (Tokuju Co. , Ltd: Mixwell blender V1-60) to obtain a molding material.

The molding material was then tableted at a tableting pressure of 9,000 N in a high-speed rotary tableting machine (Kikusui Seisakusho Ltd.: VIRGO 12) with a punch selected so that the diameter of the tablet is 7 mm in the form described in Fig. 2, and each of R (curvature radius), S (curvature radius), a, b, c, and d takes the values shown in table 1 while adjusting the thickness of such tablets, thereby to prepare dividable tablets according to the present invention.

### Test examples

The evaluation results of the dividable tablet of the present invention are shown in the following table.

The sensory test in the table was performed by five panelists selected at random, each panelist dividing the tablet on a flat table by pressing with finger with the dividing line facing downward and evaluating the easiness of dividing based on four stages (easy to divide: 3 points, relatively easy to divide: 2 points, not easy to divide: 1 point, no dividing: 0 point), where 15 points on a 15 point scale being ++, 10 to 14 points as +, 5 to 9 points as ±, and less than or equal to 4 points as -. The evaluation of o in which most of the panelists answered as easy to divide is preferable, and the evaluation of + in which everyone answered as easy to divide is most preferable.

The CV(%) after dividing, in the table, is the percentage of the standard deviation divided by the average value of the weight of 20 half-tablets obtained by dividing 10 tablets, and a lower CV(%) indicates more precise division of tablets, wherein the value of 2 to 4 is preferable and the value of less than or equal to 2 is extremely preferable.

**Table 1**

| No. | R (mm) | S (mm) | a (°) | b (°) | c (mm) | d (mm) | c:d | Dividing line facing downward | |
|---|---|---|---|---|---|---|---|---|---|
| | | | | | | | | Sensory evaluation | CV (%) after dividing |
| 1 | 12 | 5 | 60 | 72 | 3.40 | 2.08 | 1:0.61 | ± | 2.1 |
| 2 | 12 | 5 | 60 | 73 | 3.30 | 1.98 | 1:0.60 | + | 2.5 |
| 3 | 12 | 5 | 60 | 77 | 3.05 | 2.00 | 1:0.66 | ± | 1.5 |
| 4 | 12 | 5 | 60 | 76 | 3.13 | 1.94 | 1:0.62 | + | 1.8 |
| 5 | 12 | 5 | 60 | 76 | 3.14 | 1.82 | 1:0.58 | + | 2.4 |
| 6 | 12 | 5 | 60 | 78 | 3.00 | 1.95 | 1:0.65 | + | 2.2 |
| 7 | 12 | 5 | 60 | 78 | 3.00 | 1.68 | 1:0.56 | ++ | 3.0 |
| 8 | 12 | 5 | 60 | 79 | 2.98 | 1.74 | 1:0.60 | ++ | 2.0 |
| 9 | 12 | 5 | 60 | 81 | 2.87 | 1.55 | 1:0.54 | ++ | 3.2 |
| 10 | 12 | 5 | 60 | 82 | 2.83 | 1.64 | 1:0.58 | ++ | 1.8 |
| 11 | 12 | 5 | 60 | 83 | 2.75 | 1.43 | 1:0.52 | ++ | 3.4 |
| 12 | 12 | 5 | 60 | 84 | 2.70 | 1.51 | 1:0.56 | ++ | 1.4 |
| 13 | 12 | 5 | 60 | 86 | 2.64 | 1.32 | 1:0.50 | ++ | 2.6 |
| 14 | 12 | 5 | 60 | 87 | 2.59 | 1.40 | 1:0.54 | ++ | 1.7 |
| 15 | 12 | 5 | 60 | 89 | 2.48 | 1.29 | 1:0.52 | ++ | 1.8 |
| 16 | 12 | 5 | 60 | 92 | 2.38 | 1.19 | 1:0.50 | ++ | 1.9 |

As apparent from the table, easy and accurate dividing was achieved in all the cases when the tablet is divided by being pressed with finger with the dividing line facing downward on the flat surface. In particular, more easy and accurate dividing was achieved when the ratio c/d of c and d is 1/0.60 s c/d ≤ 1/0.40 in the case where b is greater than or equal to 70° and less than 75°; when the ratio c/d of c and d is 1/0.65 ≤ c/d ≤ 1/0.40 in the case where b is greater than or equal to 75° and less than 78°; and when the ratio c/d of c and d is 1/0.70 s c/d s 1/0.40 in the case where b is greater than or equal to 78°. More easy and accurate dividing is possible when b is greater than or equal to 78° and the ratio c/d of c and d is 1/0.70 ≤ c/d ≤ 1/0.40.

Easy and accurate dividing was also achieved in all cases when the tablet is divided by being pressed with finger with the dividing line facing upward on the flat surface.

### Industrial Applicability

According to the present invention, there is provided the dividable tablet that is divided by being sandwiched between two flat plates, followed by pressing, that is divided by being pressed with finger and the like with the dividing line facing downward on the flat surface, that is divided through similar methods even from the outside the packaging such as PTP, and preferably, that is divided regardless of whether the upper or the lower surface of the tablet is facing upwards, that is further easily and accurately divided, and that has superior strength.

## Claims

1. A dividable tablet whose lower surface is a curved surface gradually rising from a central part exactly below a dividing line and whose upper surface is divided into two or more surfaces by at least one dividing line formed by U-shaped groove, or V-shaped groove having a center angle of less than or equal to 105°; wherein, in a side view of the tablet seen from the extended line of the dividing line, in a virtual triangle connecting a center of the lower surface and any two points on each of the two or more upper surfaces having the farthest distance from a tangent line to the center of the lower surface, the angle at the center of the lower surface is greater than or equal to 70°, a ratio c/d of a distance (c) between any one of the points on each of the two or more upper surfaces having the farthest distance from the tangent line to the center of the lower surface and the tangent line to the center of the lower surface and a distance (d) between a lowest part of the dividing line and the center of the lower surface is 1/0.70 ≤ c/d s 1/0.40, and (i) when c/d is in the range of 1/0.60 ≤ c/d ≤ 1/0.40, the angle at the center of the lower surface of the corresponding virtual triangle is greater than or equal to 70°, (ii) when c/d is in the range of 1/0.65 ≤ c/d < 1/0.60, the angle at the center of the lower surface of the corresponding virtual triangle is greater than or equal to 75°, and (iii) when c/d is in the range of 1/0.70 ≤ c/d < 1/0.65, the angle at the center of the lower surface of the corresponding virtual triangle is greater than or equal to 70° and less than 75°, or greater than or equal to 78°.

2. The dividable tablet according to claim 1, wherein (i) the angle at the center of the lower surface of the virtual triangle is greater than or equal to 70° and c/d is 1/0.60 ≤ c/d ≤ 1/0.40, (ii) the angle at the center of the lower surface of the virtual triangle is greater than or equal to 75° and c/d is 1/0.65 ≤ c/d ≤ 1/0.40, or (iii) the angle at the center of the lower surface of the virtual triangle is greater than or equal to 78° and c/d is 1/0.70 ≤ c/d ≤ 1/0.40.
